# FASCICULE DE BREVET EUROPEEN

(11) **EP 2 654 925 B1**
(45) Date de publication et mention de la délivrance du brevet: **19.11.2014**
(21) Numéro de dépôt: 11802946.1
(22) Date de dépôt: 21.12.2011
(51) Int. Cl.: B01D 53/04

(54) **PROCÉDÉ D'ÉPURATION FINALE DE BIOGAZ**
ABSCHLIESSENDES REINIGUNGSVERFAHREN FÜR BIOGAS
FINAL BIOGAS PURIFICATION PROCESS

(30) Priorité: 21.12.2010 FR 1060988
(43) Date de publication de la demande: 30.10.2013
(73) Titulaire: L'Air Liquide Société Anonyme pour l'Etude et l'Exploitation des Procédés Georges Claude, 75007 Paris (FR)
(72) Inventeur: BERNHARDT, Jean-Marc, 38500 La Buisse (FR); BRIEND, Pierre, 38170 Seyssinet (FR); GRILLOT, David, 38140 Rives (FR); RENOU, Elise, 75015 Paris (FR); SAULQUIN, Simon, 38000 Grenoble (FR); WEITTEN, Olivier, 38100 Grenoble (FR)
(74) Mandataire: Conan, Philippe Claude
(86) Numéro de dépôt international: PCT/EP2011/073655
(87) Numéro de publication internationale: WO 2012/085128

(56) Documents cités:
- EP-A2- 1 291 067
- FR-A- 818 151
- US-A1- 2008 289 497
- US-A1- 2008 314 245
- US-A1- 2011 041 689
- US-B1- 6 508 862

## Description

La présente invention est relative à un procédé d'épuration par adsorption d'un débit d'alimentation riche en méthane et contenant au moins du dioxyde de carbone.

Elle concerne en particulier l'épuration finale de biogaz, dans le but de produire du méthane, de préférence liquide ; en effet, la liquéfaction est un mode de conditionnement du méthane qui permet de le stocker et le transporter économiquement.

La fermentation anaérobie (sans oxygène) de déchets organiques dégage un gaz constitué essentiellement de méthane et de dioxyde de carbone, appelé biogaz. Ce processus est en plein développement, à la fois pour limiter les émissions de gaz à effet de serre dans l'atmosphère mais aussi pour exploiter le biogaz ainsi produit qui est une source d'énergie appréciable.

Le biogaz est en particulier destiné à alimenter des turbines électriques ou à servir comme carburant pour les véhicules.

Issu de la fermentation anaérobie, le biogaz contient du CO₂ et du CH₄, dans des proportions respectives dépendant de la nature des matières fermentées, en général, le biogaz produit contient entre 55 et 65% de méthane.

Le biogaz, une fois épuré de son dioxyde de carbone, de son eau et de son hydrogène sulfuré H₂S peut être valorisé en tant que methane, en particulier comme carburant.

Ainsi que rappelé ci-dessus, la liquéfaction est un mode préféré de conditionnement du méthane, que ce soit à des fins de stockage ou de transport. Toute unité d'épuration de biogaz contenant du CO₂, nécessitera donc d'inclure à son procédé une purification finale de méthane pour éliminer entre autres constituants ceux qui sont incompatibles avec la liquéfaction ou un autre traitement nécessitant un passage à des températures cryogéniques. Il conviendra en particulier de limiter la concentration du CO₂ à une teneur maximale in fine inférieure à 100ppm.

L'épuration finale de méthane issu du biogaz dans le but de produire du méthane liquide fait appel à différents procédés connus de l'état de l'art, qui sont l'adsorption par modulation de pression (pressure swing adsorption en langue anglaise ou PSA), l'adsorption par modulation de température (temperature swing adsorption en langue anglaise ou TSA), ou le lavage aux amines.

Ces techniques habituelles d'adsorption font appel pour la régénération des adsorbants à de grandes quantités de gaz. Cependant, les sites de production de biogaz (digesteur, décharges, ...), ne disposent en général pas de grande quantités de gaz propres (N₂, CH₄ pur) pour la régénération des adsorbeurs.

Par ailleurs, lorsque la quantité de CO₂ dans le biogaz est significative, (>1%), l'exo thermicité de l'adsorption chauffe l'adsorbant, dégradant ainsi sa capacité d'adsorption, et il est donc essentiel de disposer d'un refroidissement efficace ; lors de la régénération de l'adsorbant, il faut au contraire apporter pour la désorption des impuretés une grande quantité de chaleur.

La circulation en boucle fermée avec réchauffage pendant la phase de régénération n'est pas efficace car le gaz circulant se charge très rapidement en impuretés (CO₂) et repartit les impuretés sur la totalité du lit d'adsorbant. Le taux résiduel d'impuretés est alors trop élevé pour atteindre une qualité de gaz compatible avec la liquéfaction (< 100 ppm). Un gaz de balayage est nécessaire.

Afin de pouvoir produire du méthane purifié en opérant l'installation en continu, on utilise de façon connue deux adsorbeurs en parallèle, l'un étant en phase d'adsorption, tandis que l'autre est en phase de désorption.

Il est connu de US2008/0289497 un système pour purifier du méthane en vue de le liquéfier, et notamment pour éliminer le CO2, utilisant trois adsorbants. Tandis que l'un est en phase d'adsorption, le second est en phase de désorption, et le troisième est refroidi ; la présence de trois adsorbeurs permettant des transferts de chaleur. FR-A-818151 divulgue un procédé d'adsorption co des transferts de chaleur lors de la phase d'adsorption et lors de la phase de désorption.

Si le système ci-dessus permet de limiter l'apport d'énergie extérieure, il nécessite cependant d'utiliser trois adsorbeurs en parallèle, ce qui génère des coûts supplémentaires par rapport à une installation classique utilisant deux adsorbeurs. Le problème qui se pose est donc de proposer une solution pour épurer du méthane impur, notamment issu du biogaz, de sorte produire du méthane de pureté compatible avec la liquéfaction, tout en limitant les coûts, à la fois en terme d'investissement et en terme d'opération - pas de troisième adsorbeur, consommations d'utilités, en particulier d'énergie réduites.

Par méthane impur, (ou débit riche en méthane), on entend du méthane d'une teneur en CO₂ inférieure à 5%, de préférence inférieure à 2%.

Par méthane purifié (ou méthane de pureté compatible avec la liquéfaction), on entend, selon l'invention du méthane présentant une teneur en dioxyde de carbone inférieure à 100ppm, de préférence inférieure à 50 ppm..

Selon un objet de l'invention, il est proposé un procédé d'épuration par adsorption d'un débit d'alimentation riche en méthane et contenant au moins du dioxyde de carbone, mettant en oeuvre deux échangeurs-adsorbeurs (Ads1, Ads2) de type tube-calandre, comprenant au moins les étapes suivantes:
1) envoyer ledit débit d'alimentation à un échangeur-adsorbeur du type tube-calandre muni d'un adsorbant dans les tubes, et d'un fluide thermique refroidissant circulant dans la calandre dudit échangeur-adsorbeur pour produire un débit épuré appauvri (au moins) en dioxyde de carbone par rapport au débit d'alimentation, puis
2) faire circuler un fluide thermique chaud dans la calandre de façon à désorber les impuretés retenues par l'adsorbant et régénérer celui-ci,
dans lequel les étapes 1) et 2) sont réalisées alternativement, sur les deux échangeurs-adsorbeurs installés en parallèle, le débit d'alimentation étant envoyé à l'un des échangeurs-adsorbeurs tandis que le second est en phase de régénération, puis au second tandis que le premier est en phase de régénération, et comprenant en outre au début de l'étape 2) une étape de réchauffage progressif de l'échangeur adsorbeur à régénérer, et en fin d'étape 2) une étape de refroidissement progressif de l'échangeur adsorbeur régénéré, ces étapes de mise en température progressive des échangeurs adsorbeurs comprenant au moins des échanges de fluide thermique entre la calandre de l'échangeur adsorbeur, au moins deux moyens de stockage (S1, S2) - de capacité de stockage comparable à la capacité de la calandre -, stockant intermédiairement le fluide thermique à des températures variables et un moyen de stockage/ chauffage (C) d'une capacité supérieure à la capacité de la calandre, apte à assurer un chauffage additionnel du fluide thermique pour la mise en oeuvre de l'étape 2).

La technologie proposée pour les deux échangeurs-adsorbeurs est du type échangeur tube-calandre contenant l'adsorbant dans les tubes et un fluide thermique circulant dans la calandre. L'avantage est de bénéficier, via la surface des tubes, d'une grande capacité d'échange de chaleur indispensable à l'alternance des régimes thermiques de fonctionnement du procédé.

L'association des deux échangeurs adsorbeurs installés en parallèle opérant pour l'un en phase d'adsorption, tandis que l'autre est en phase de régénération assure la production continue de gaz épuré. Tout au long de la phase d'adsorption selon l'étape 1, un fluide thermique froid circule dans la calandre afin de conserver une température faible dans l'adsorbant et ainsi conserver ses propriétés adsorbantes. Lorsque l'adsorbant est saturé en impuretés, il est nécessaire de procéder à la régénération de l'adsorbant. La régénération s'effectuant à chaud, la solution de l'invention permet de limiter le coût de cette régénération. En effet , la solution proposée permet de réaliser l'adsorption des impuretés en refroidissant en continu l'adsorbant de l'échangeur adsorbeur en phase d'adsorption, et de réaliser pendant le même temps la régénération de l'adsorbant du deuxième échangeur-adsorbeur, comprenant le chauffage par étapes successives de l'adsorbant - utilisant pour cela des sources de chaleur judicieusement mises à disposition de sorte à minimiser la consommation d'énergie de la régénération - jusqu'à régénération de l'adsorbant, suivi du refroidissement lui aussi par étapes successives de l'adsorbeur. Ce refroidissement selon l'invention prépare l'échangeur adsorbeur Ads2 pour l'étape d'adsorption suivante, et prépare les différents moyens de chauffage et de stockage pour l'étape de régénération appliquée à cette fois à Ads1.

Un fluide thermique préféré est de l'eau.

De préférence, le réchauffage progressif de l'échangeur adsorbeur Ads2, en début d'étape de régénération comprend au moins les étapes de : préalablement au réchauffage :
(i) mise à disposition :
   o de fluide thermique froid dans la calandre de l'échangeur adsorbeur à une température inférieure à ou de l'ordre de la température ambiante,
   o de fluide thermique chaud dans le moyen de stockage S1 à une température supérieure à 110°C,
   o de fluide thermique tiède dans le moyen de stockage S2 à une température comprise entre 70°C et 110°C, de préférence entre 80°C et 100°C
   o de fluide thermique chaud dans le moyen de stockage/ chauffage C à une température supérieure à 115°C, de préférence de l'ordre 130°C,
(ii) circulation de fluide thermique froid dans la calandre de l'échangeur-adsorbeur Ads2 en phase en phase d'adsorption selon l'étape 1) pour assurer le maintien du froid durant ladite étape 1),
Puis réchauffage progressif par :
(iii) échange, en fin d'étape 1) du fluide thermique froid présente dans la calandre de Ads2 avec le fluide tiède contenu dans le moyen de stockage S2 ; S2 contient alors du fluide froid,
(iv) réchauffage du fluide contenu dans la calandre de Ads2 par échange de chaleur via un échangeur **12** jusqu'à une température de fluide tiède comprise entre 80°C et 105°C, de préférence de l'ordre de 100°C,
(v) échange de fluide thermique tiède contenu dans la calandre de Ads2 à l'issue de l'étape (iv) avec le fluide chaud contenu dans le moyen de stockage S1,
(vi) réchauffage du fluide thermique chaud présent dans la calandre de Ads2 par circulation de fluide entre la calandre et le moyen de stockage/ chauffage (C) jusqu'à la fin de la régénération selon l'étape 2).

Pendant la phase de régénération, un fluide thermique chaud - préférentiellement de l'eau surchauffée à une température supérieure à 110°, plus préférentiellement de l'ordre de 130°C - circule ainsi dans la calandre de l'adsorbeur en phase de régénération afin d'apporter la chaleur nécessaire à la désorption des impuretés.

La fin de la phase de régénération de l'adsorbant d'Ads2 marque la fin de la circulation de fluide chaud entre Ads2 et le ballon C.

L'utilisation d'un moyen de chauffage HX annexe afin de réchauffer modérément le fluide thermique dans la calandre lors de l'étape (iv) est rendue nécessaire pour compenser la baisse de température induite par le réchauffage de l'échangeur-adsorbeur.

De préférence, le refroidissement progressif de l'échangeur adsorbeur régénéré Ads2 à l'issue de l'étape 2) et préalablement à l'adsorption selon l'étape 1) comprend au moins les étapes de :
préalablement au refroidissement :
   (vii) mise à disposition de :
      o de fluide thermique chaud dans la calandre de l'échangeur adsorbeur Ads2, à une température de fluide chaud supérieure à 110°C, de préférence entre 115°C et 130°C,
      o de fluide thermique dans le moyen de stockage S1 à une température d'eau de l'ordre de 100°C à 110°C
      o du fluide thermique froid dans le moyen de stockage S2,
      o du fluide thermique chaud dans le moyen de stockage/ chauffage (C) à une température supérieure à 115°C, de préférence de l'ordre de 130°C,
      et refroidissement progressif incluant
   (viii) échange du fluide thermique présent dans la calandre de Ads2 avec le fluide thermique du moyen de stockage S1 ; S1 contient alors du fluide thermique chaud,
   (ix) échange du fluide tiède contenu dans Ads2 avec le fluide froid contenu dans le moyen de stockage S2,
   (x) réchauffage du fluide contenu dans S2 par HX de sorte à reconstituer le stockage tiède selon l'étape (i),
   (xi) refroidissement continu de Ads2 par du fluide thermique froid.

Selon l'invention, les mises en température des échangeurs-adsorbeurs entre les étapes d'adsorption et de régénération, c'est-à-dire le chauffage de l'échangeur en fin d'adsorption, et le refroidissement de l'échangeur en fin de régénération s'effectuent de manière progressive, par étapes successives d'échange de fluide entre les calandres et au moins deux moyens de stockage d'une capacité de stockage comparable à la capacité des calandres, et un moyen de stockage/ chauffage (C) d'une capacité très supérieure, de préférence au moins double de la capacité de la calandre, apte à assurer un chauffage additionnel de fluide thermique pour la mise en oeuvre de l'étape 2). Les moyens de stockage stockent intermédiairement le fluide thermique (de préférence de l'eau) à des températures variables -l'un des moyens de stockage stockant alternativement de l'eau froide et de l'eau tiède (soit à une température comprise entre 80°C et 100°C) et le deuxième stockage stockant alternativement de l'eau tiède (soit à une température comprise entre 80°C et 100°C) et de l'eau chaude (soit à une température entre 100°C et 130°C) -. Le moyen de stockage/chauffage est de préférence un cumulus (chauffe-eau) additionnel, relié à l'adsorbeur-échangeur en phase de régénération, il assure le maintien de la température d'eau chaude dans la calandre durant l'étape 2) de régénération.

Selon des variantes préférées de l'invention, celle-ci peut concerner un procédé dans lequel :
Durant l'étape de régénération, les impuretés sont avantageusement retirées de l'adsorbant par pompage sous vide, à une pression comprise de préférence entre 100 à 200 mbara de sorte à associer une modulation de pression à la modulation de température.

L'adsorption étant réalisée préférentiellement à une pression comprise entre 7 et 15 bars, pour passer du mode adsorption au mode régénération il faut dépressuriser l'échangeur-adsorbeur, afin d'augmenter l'impact de la réduction de pression sur la régénération, un gaz inerte, en général de l'azote, peut être injecté à l'opposé de la pompe à vide de sorte à diluer la fraction d'impureté dans le gaz désorbé et réduire ainsi sa pression partielle.

Avantageusement, la remise en pression de l'échangeur-adsorbeur est réalisée en même temps que le refroidissement, facilitant ainsi le refroidissement.

Le fluide froid est préférentiellement prélevé sur le système de refroidissement général du site.

Le moyen de stockage/chauffage C est avantageusement chauffé par une résistance électrique de chauffage.

Le fluide thermique sortant des différents moyens de stockage est mis en circulation via des pompes de circulation et/ ou est poussé avec le fluide thermique entrant dans les dits stockages.

La source extérieure chaufant le ballon de stockage / chauffage C peut être une résistance électrique intégrée au circuit afin d'apporter le complément de chaleur nécessaire à la régénération pendant un temps suffisant pour assurer le réchauffage complet de l'adsorbant par conduction, compenser les pertes de chaleur dans le gaz désorbé et apporter la chaleur nécessaire à la désorption (transfert de matière endothermique).

Ainsi, avantageusement, une partie de la chaleur nécessaire à la régénération provient d'un réservoir de fluide thermique équipé d'une résistance électrique de chauffage.

Dans le cas où le méthane purifié est liquéfié, un apport d'énergie complémentaire est obtenu par échange de chaleur avec le gaz de cycle du liquéfacteur dans un étage de l'échangeur final du compresseur qui permettra une augmentation conséquente de la température. Cet apport de chaleur est utilisé entre autres dans l'étape (iv) ; l'échangeur y est identifié en tant qu'élément **12 .**

L'invention va maintenant être décrite en se référant à un exemple de mise en oeuvre de l'invention, et aux figures jointes, dont :
- la Figure 1 représente schématiquement une installation d'épuration de biogaz selon l'invention,
- les Figures 2a à 2g schématisent des étapes de chauffage et de refroidissement de l'échangeur -adsorbeur Ads2 de l'installation de la Figure 1.

Les nombres en gras dans le texte ci-après reprennent les nombres de référence des éléments dans les figures ; pour une meilleure compréhension du déroulement du procédé, les éléments peuvent aussi être identifiés par des pouvant associer des lettres et des chiffres. La correspondance entre les deux modes d'identification est spécifiée dans le texte ci-dessous.

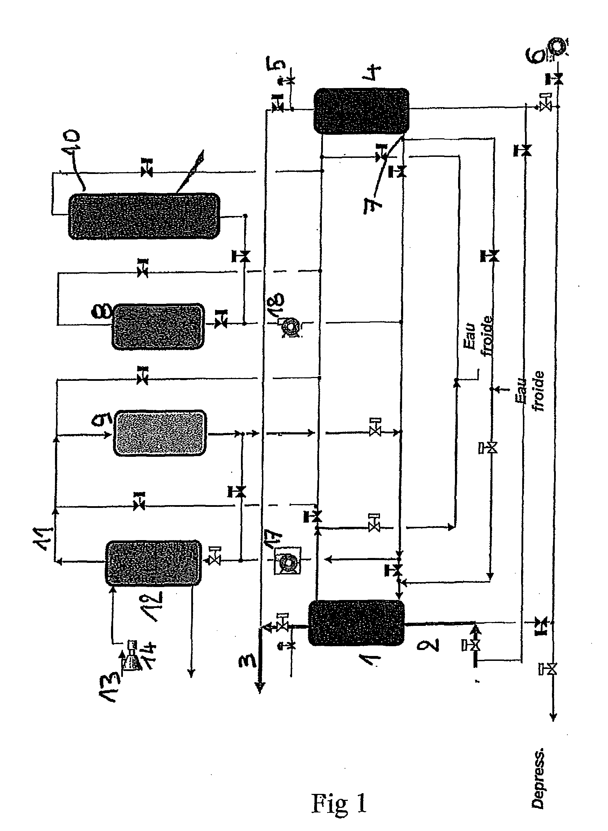

L'installation de la Figure 1 fonctionne de la manière suivante :
- L'échangeur-adsorbeur Ads2 ou Ads1(respectivement **1** ou **4)** est, lorsqu'il est en mode adsorption, alimenté en gaz impur **2** et produit du gaz pur **3 ;** la régénération de l'autre échangeur adsorbeur s'effectue durant la phase d'adsorption du premier.
- Ads1 et Ads 2 peuvent être alimentés :
   o en azote gazeux GN2 d'élution **5,** le débit désorbé est pompé par la pompe à vide **6,**
   o en eau à température variable provenant du bac S1 **8,** du bac S2 **9** et du moyen de stockage/ chauffage **10,**
   o en eau chaude **11** provenant de l'échangeur HX **12** qui échangeavec le gaz GN2 chaud **13** comprimé dans le compresseur du cycle de liquéfaction **14** (cycle de liquéfaction de méthane non représenté),
   o en eau froide **15** en provenance du circuit de refroidissement de l'installation,
- des pompes à eau de circulation **17** et **18** permettant les transferts d'eau,
- en outre, (non représentés) un circuit permet la dépressurisation de l'adsorbeur-échangeur en début de régénération, et un circuit séparé permet la repressurisation en gaz process de l'adsorbeur régénéré.

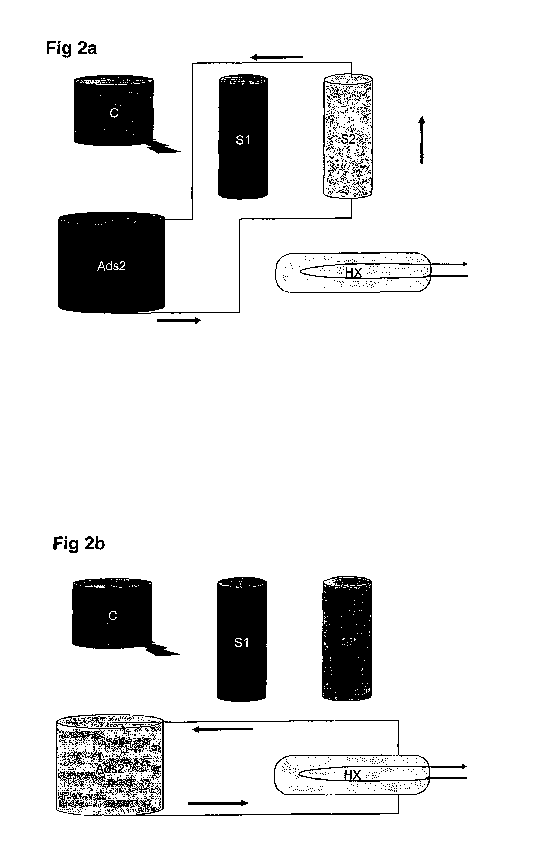

Un exemple d'application de l'invention est décrit ci-après en liaison avec la Figure 1 et les Figures 2a à 2g:
L'adsorption se fait par alternance dans les deux échangeurs-adsorbeurs **1** et **4** (de type tube et calandre, il est impératif de les faire fonctionner par circulation d'eau à températures contrôlées dans la calandre)

Le procédé de régénération selon l'invention est conçu pour limiter la consommation d'énergie électrique de **10** (ballon C).

Le principe proposé consiste à mettre en oeuvre :
- le ballon C qui stocke 2m³ d'eau et réchauffe électriquement l'eau de 110°C à 130°C, environ ;
- une autre source de chaleur qui est le gaz **13** en sortie d'un compresseur **14.** Le gaz **13** est utilisé dans l'échangeur precooler **12** (HX) pour réchauffer de l'eau et obtenir de l'eau tiède jusqu'à 100°C ;
- le bac **8** d'eau chaude (S1) ; il stocke 1m³ alternativement d'eau chaude (110°C à 120°C) ou d'eau tiède (≈ 100°C) ;
- le bac **9** d'eau tiède (S2), il stocke alternativement de l'eau tiède, qui a été réchauffée par HX à 100°C ou de l'eau froide à environ 30° provenant de Ads1 ou Ads2 après adsorption ;

Les étapes de mise progressive en température sont décrites ci-dessous pour Ads2, elles sont semblables pour Ads1.
La Figure 2a schématise l'étape iii du procédé durant laquelle Ads2 échange 0,9m3 d'eau à 20°C contenue dans la calandre avec 0,9m3 d'eau à 100°C contenue dans S2. Il s'agit là de la 1^{ère} étape de préchauffage de l'adsorbeur par l'eau tiède de S2 (l'eau initialement présente dans S2 est l'eau issue du cycle de régénération précédent, c'est-à-dire de celui de Ads1). La quantité de chaleur transférée à Ads2 contribue essentiellement au réchauffage des matériaux de construction de l'échangeur-adsorbeur, le transfert de chaleur est très efficace car la diffusivité thermique de l'acier est très élevée. ; l'eau dans Ads2 est alors à 50°C environ.
La Figure 2b schématise l'étape iv du procédé durant laquelle de l'eau circule en boucle entre Ads2 et HX (l'eau contenue initialement dans HX est à une température d'eau tiède de l'ordre de 100°C selon l'étap i).. L'eau de Ads2 est ainsi portée à 90°C environ.

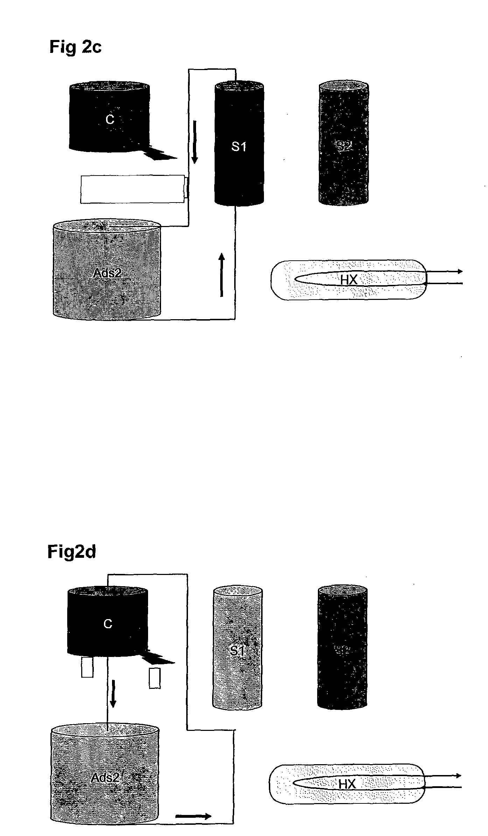

La Figure 2c schématise l'étape v du procédé durant laquelle l'eau tiède contenue dans la calandre de Ads2 (90°C) est échangée avec l'eau chaude stockée dans S1 (l'eau initialementcontenue dans 8 (S1) est à une température d'eau chaude de l'ordre de 110°C selon l'étape i).
La figure 2d schématise l'étape vi du procédé durant laquelle de l'eau chaude circule de 10 (ballon C) vers la calandre de Ads2, simultanément , l'eau intialement contenue dans Ads2 ; simultanément, l'eau chassée de Ads2 circule jusqu'à C. Afin d'assurer un réchauffage optimal de l'eau dans la calandre, le volume circulé est supérieur au volume de la calandre (selon l'exemple1,4m3 d'eau à 130°C circule pour un volume de la calandre de l'ordre de 0,9m3).

La fin de la phase de régénération de l'adsorbant d'Ads2 est conjointe avec la fin de réchauffage d'Ads2 par le ballon C.

Les étapes suivantes ont pour but de refroidir l'eau contenue dans la calandre d'Ads 2 de sorte à ce qu'il soit thermiquement opérationnel pour l'étape d'adsorption du cycle suivant.

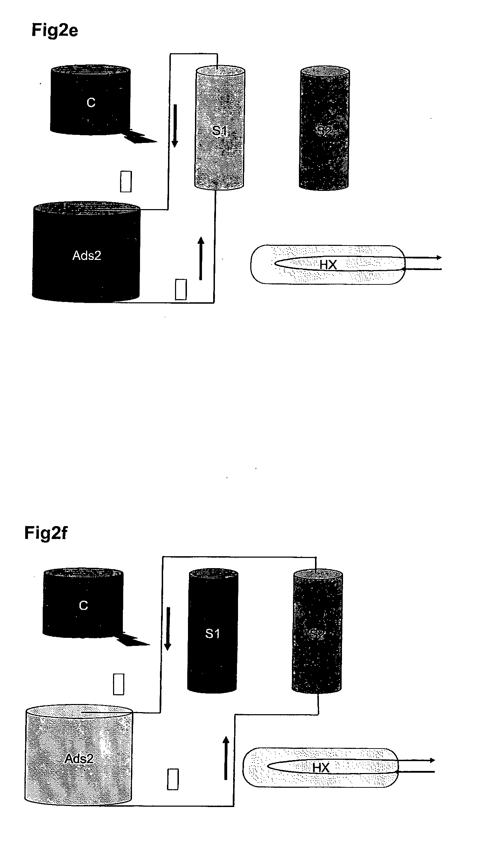

La figure 2e schématise ainsi l'étape vii du procédé durant laquelle l'eau chaude d'Ads2 (à environ 120°C) est permutée avec l'eau tiède de S1 (à environ 100°C). Cette étape permet ainsi de refroidir l'adsorbeur en conservant - dans le stockage S1 - l'eau chaude qu'il contenait pour les besoins internes au procédé...

La figure 2f schématise l'étape viii du procédé durant laquelle l'eau tiède d'Ads1 est permutée avec l'eau froide de S2 ; permettant ainsi de poursuivre le refroidissement de l'adsorbeur/échangeur Ads2 en conservant - dans le stockage S2 - l'eau tiède qu'il contenait pour les besoins internes au procédé. Dans l'étape R6, pour conserver l'énergie contenue dans l'eau tiède, elle est permutée avec l'eau du Bac-2 qui contenait de l'eau froide du précédent transfert.

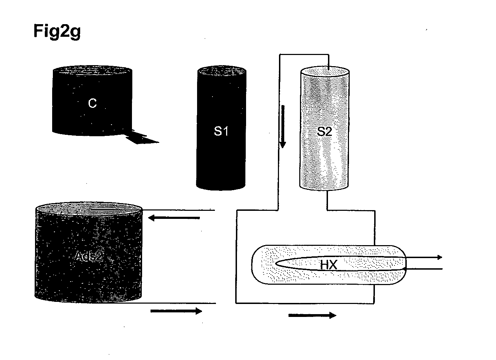

Enfin, la Figure 2g schématise l'étape ix de refroidissement final d'Ads2 qui s'obtient par circulation d'eau froide. Pendant cette étape, l'eau du ballon S2 est réchauffée par circulation en boucle dans HX.

En même temps que se déroule la phase de régénération de **1** (Ads2) qui fait appel aux moyens **8, 9, 10, 12** - la phase de régénération comprenant les étapes de réchauffage, la régénération et le refroidissement -, l'adsorbeur **4** (Ads1) est en phase d'adsorption. La fin de la régénération d'Ads2 coïncide avec la fin de l'adsorption sur Ads1 ; on procède alors, selon le procédé de l'invention à la régénération d'Ads1 tandis qu'Ads1 est en phase d'adsorption.

Deux essais ont été réalisés à des fins de comparaison, mettant en oeuvre les éléments suivants :
- éléments communs :
   ∘ 2 échangeurs/adsorbeurs de type calandre/ tube, à une passe, présentant un volume de calandre de 0,88m3 dans laquelle circule de l'eau ;
   ∘ 1 échangeur pre-cooler HX de type calandre /tube, à 2 passes, présentant un volume de calandre de 0,3m3 dans la quelle circule l'eau ;
   ∘ 1 bac S de stockage d'eau d'un volume de 1m3 ;
   ∘ 1 un « cumulus» C pour le stockage et le chauffage d'un volume d'eau de 2 m3
- pour l'essai selon l'invention, un second bac de stockage de type S est ajouté.

Les essais réalisés ont montré que l'énergie consommée pour chauffer à 130°C l'eau du ballon C est de 68kW lorsqu'un seul bac S est utilisé, tandis que l'ajout d'un bac intermédiaire supplémentaire de même type S permet de réduire la consommation à 23,3kW (soit d'un facteur 3 environ).

L'invention a été décrite dans le cas où le fluide thermique est de l'eau, ceci a permis de préciser certaines données, domaines de températures lors des étapes de chauffage et de refroidissement en particulier, mais l'utilisation d'autres fluides est envisageable, en fonction des techniques d'adsorption et de régénération mises en oeuvre, étant entendu que le principe de l'invention réside dans :
- l'utilisation de deux échangeurs adsorbeurs de type tube calandre en alternance,
- l'utilisation d'un moyen de stockage tiède/ froid et d'un moyen de stockage tiède/ chaud en plus du moyen de stockage/chauffage C, pour assurer le chauffage et le refroidissement de l'adsorbeur en régénération.

L'invention utilise astucieusement la rapidité de la régénération à haute température comparativement à l'adsorption, qui donne la possibilité de procéder aux circulations de fluides thermiques à différentes températures et aux chauffages successifs de l'invention.

Les avantages de l'invention sont multiples :
L'utilisation de deux échangeurs tube-calandre avec un adsorbant dans les tubes et un fluide thermique à l'extérieur auquel on fait appel pendant l'adsorption et la régénération, combinée avec l'utilisation de 2 stockages intermédiaires stockant astucieusement de l'eau à des températures variables présente ainsi de nombreux avantages, parmi lesquels :
   - la possibilité d'utiliser de l'eau en tant que fluide caloporteur, l'eau de refroidissement étant en général largement disponible sur site.
   - la limitation de la consommation d'énergie électrique nécessaire à la régénération via de stockages appropriés de fluide thermique (de l'eau tiède et de l'eau chaude à deux températures différentes), via aussi l'utilisation d'un étage de l'échangeur final de compression du gaz de cycle du liquéfacteur de biogaz.

## Revendications

1. Procédé d'épuration par adsorption d'un débit d'alimentation riche en méthane et contenant au moins du dioxyde de carbone, mettant en oeuvre deux échangeurs-adsorbeurs (Ads1, Ads2) de type tube-calandre, comprenant au moins les étapes suivantes:
1) envoyer ledit débit d'alimentation à un échangeur-adsorbeur du type tube-calandre muni d'un adsorbant dans les tubes, et d'un fluide thermique refroidissant circulant dans la calandre dudit échangeur-adsorbeur pour produire un débit épuré appauvri (au moins) en dioxyde de carbone par rapport au débit d'alimentation, puis
2) faire circuler un fluide thermique chaud dans la calandre de façon à désorber les impuretés retenues par l'adsorbant et régénérer celui-ci,
dans lequel les étapes 1) et 2) sont réalisées alternativement, sur les deux échangeurs-adsorbeurs installés en parallèle, le débit d'alimentation étant envoyé à l'un des échangeurs-adsorbeurs tandis que le second est en phase de régénération, puis au second tandis que le premier est en phase de régénération, **caractérisé en ce qu'**il comprend en outre au début de l'étape 2) une étape de réchauffage progressif de l'échangeur adsorbeur à régénérer, et en fin d'étape 2) une étape de refroidissement progressif de l'échangeur adsorbeur régénéré, ces étapes de mise en température progressive des échangeurs adsorbeurs comprenant au moins des échanges de fluide thermique entre la calandre de l'échangeur adsorbeur, au moins deux moyens de stockage (S1, S2) - de capacité de stockage comparable à la capacité de la calandre -, stockant intermédiairement le fluide thermique à des températures variables et un moyen de stockage/ chauffage (C) d'une capacité supérieure à la capacité de la calandre, apte à assurer un chauffage additionnel du fluide thermique pour la mise en oeuvre de l'étape 2).

2. Procédé selon la revendication 1 dans lequel le fluide thermique est de l'eau.

3. Procédé selon la revendication 1 ou la revendication 2 dans lequel le réchauffage progressif du deuxième échangeur adsorbeur (Ads2) en début d'étape de régénération comprend au moins les étapes de :
préalablement au réchauffage : le dexième moyen de stockage (S2)
(i) mise à disposition de :
o de fluide thermique froid dans la calandre de l'échangeur adsorbeur à une température inférieure à ou de l'ordre de la température ambiante,
o de fluide thermique chaud dans le premier moyen de stockage (S1) à une température supérieure à 110°C,
o de fluide thermique tiède dans le deuxième moyen de stockage (S2) à une température comprise entre 70°C et 110°c, de préférence entre 80°C et 100°C
o de fluide thermique chaud dans le moyen de stockage/ chauffage à une température supérieure à 115°C, de préférence de l'ordre 130°C,
(ii) circulation fluide thermique froid dans la calandre du deuxième échangeur-adsorbeur (Ads2) en phase d'adsorption selon l'étape 1) pour assurer le maintien du froid durant ladite étape 1),
réchauffage progressif par :
(iii) échange, en fin d'étape 1) de fluide thermique froid présent dans la calandre du deuxième échangeur-adsorbeur (Ads2) avec du fluide thermique tiède contenu dans le deuxième moyen de stockage (S2) ; le deuxième moyen de stockage (S2) contient alors du fluide thermique froid,
(iv) réchauffage du fluide thermique contenu dans la calandre du deuxième échangeur-adsorbeur (Ads2) par échange de chaleur via un échangeur (**12**) jusqu'à une température de fluide thermique tiède comprise entre 80°C et 105°C, de préférence de l'ordre de 100°C,
(v) échange du fluide thermique tiède contenu dans la calandre du deuxième échangeur-adsorbeur (Ads2) à l'issue de l'étape (iv) avec le fluide thermique chaud contenu dans le premier moyen de stockage (S1),
(vi) réchauffage du fluide thermique chaud présent dans la calandre du deuxième échangeur-adsorbeur (Ads2) par circulation de fluide thermique entre la calandre et le moyen de stockage/ chauffage (C) jusqu'à la fin de la régénération selon l'étape 2).

4. Procédé selon la revendication 3 dans lequel le refroidissement progressif du premier échangeur-adsorbeur régénéré (Ads1), à l'issue de l'étape 2) et préalablement à l'adsorption selon l'étape 1) comprend au moins les étapes de :
préalablement au refroidissement :
(vii) mise à disposition de :
o de fluide thermique chaud dans la calandre du deuxième échangeur adsorbeur (Ads2), à une température de fluide chaud supérieure à 110°C, de préférence entre 115°C et 130°C,
o de fluide thermique dans le premier moyen de stockage (S1) à une température d'eau de l'ordre de 100°C à 110°C
o du fluide thermique froid dans le deuxième moyen de stockage (S2)
o du fluide thermique chaud dans le moyen de stockage/ chauffage (C) à une température supérieure à 115°C, de préférence de l'ordre de 130°C,
et refroidissement progressif incluant
(viii) échange du fluide thermique présent dans la calandre du deuxième échangeur-adsorbeur (Ads2) avec le fluide thermique du premier moyen de stockage (S1); celui-ci (S1) contient alors de l'eau chaude,
(ix) échange du fluide tiède contenu dans le deuxième échangeur-adsorbeur (Ads2) avec le fluide froid contenu dans le deuxième moyen de stockage (S2)
(x) réchauffage de l'eau contenue dans le deuxième moyen de stockage (S2) par un moyen de chauffage HX de sorte à reconstituer le stockage d'eau tiède selon l'étape (i)
(xi) refroidissement continu de Ads2 par du fluide thermique froid.

5. Procédé selon l'une des revendications 1 à 4 dans lequel le fluide froid est prélevé sur le système de refroidissement général du site.

6. Procédé selon l'une des revendications précédentes **caractérisé en ce que** durant l'étape 2), les impuretés sont retirées de l'adsorbant par pompage sous vide de sorte à associer une modulation de pression à la modulation de température.

7. Procédé selon la revendication 6 dans lequel un gaz inerte est injecté à l'opposé de la pompe à vide de sorte à diluer la fraction d'impureté dans le gaz désorbé et à réduire ainsi sa pression partielle.

8. Procédé selon la revendication 6 ou la revendication 7 dans lequel l'adsorption est réalisée à une pression comprise entre 7 et 15 bars, et la remise en pression de l'échangeur-adsorbeur est réalisée en même temps que le refroidissement.

9. Procédé selon l'une des revendications 1 à 8 dans lequel le moyen de stockage / chauffage (C) est chauffé par une résistance électrique de chauffage.

## Patentansprüche

1. Verfahren zur Reinigung einer Beschickungsleistung, die reich an Methan ist und mindestens Kohlendioxid enthält, durch Adsorption, wobei das Verfahren zwei Tauscher-Adsorber (Ads1, Ads2) des Typs Rohrbündeltauscher verwendet und mindestens die folgenden Schritte umfasst:
1) Leiten der Beschickungsleistung zu einem Tauscher-Adsorber des Typs Rohrbündeltauscher, der mit einem Adsorptionsmittel in den Rohren und einem kühlenden thermischen Fluid versehen ist, das in dem Bündel des Tauscher-Adsorbers im Umlauf ist, um eine gereinigte Leistung herzustellen, die gegenüber der Beschickungsleistung (mindestens) an Kohlendioxid abgereichert ist, und anschließend
2) Inumlaufbringen eines heißen thermischen Fluids in dem Bündel, derart, dass die Verunreinigungen, die in dem Adsorptionsmittel zurückgehalten wurden, desorbiert werden und dieses regeneriert wird,
bei dem die Schritte 1) und 2) an den zwei Tauscher-Adsorbern, die parallel angeordnet sind, alternierend durchgeführt werden, wobei die Beschickungsleistung zu einem der Tauscher-Adsorber geleitet wird, während der zweite in der Regenerationsphase ist, und sie anschließend zu dem zweiten geleitet wird, während der erste in der Regenerationsphase ist, **dadurch gekennzeichnet, dass** das Verfahren zu Beginn von Schritt 2) einen Schritt des progressiven Erhitzens des Tauscher-Adsorbers, der zu regenerieren ist, und am Ende von Schritt 2) einen Schritt des progressiven Abkühlens des regenerierten Tauscher-Adsorbers enthält, wobei diese Schritte der progressiven Temperierierung der Tauscher-Adsorber mindestens den Austausch von thermischem Fluid zwischen dem Bündel des Tauscher-Adsorbers, mindestens zwei Speichermittel (S1, S2) - deren Speicherkapazität mit der Kapazität des Bündels vergleichbar ist -, die das thermische Fluid bei variablen Temperaturen zwischenspeichern, und ein Speicher-/Erhitzungs-Mittel (C) umfassen, dessen Kapazität höher ist als die Kapazität des Bündels und das in der Lage ist, ein zusätzliches Erhitzen des thermischen Fluids bei der Durchführung von Schritt 2) sicherzustellen.

2. Verfahren nach Anspruch 1, bei dem das thermische Fluid Wasser ist.

3. Verfahren nach Anspruch 1 oder Anspruch 2, bei dem das progressive Erhitzen des zweiten Tauscher-Adsorbers (Ads2) am Anfang des Regenerationsschritts mindestens die folgenden Schritte umfasst:
vor dem Erhitzen:
(i) Bereitstellen:
o von kaltem thermischem Fluid in dem Bündel des Tauscher-Adsorbers mit einer Temperatur, die niedriger als die oder in der Größenordnung der Umgebungstemperatur ist,
o von heißem thermischem Fluid in dem ersten Speichermittel (S1) mit einer Temperatur, die höher als 110 °C ist,
o von lauwarmem thermischem Fluid in dem zweiten Speichermittel (S2) mit einer Temperatur, die zwischen 70 °C und 110 °C, bevorzugt zwischen 80 °C und 100 °C beträgt,
o von heißem thermischem Fluid in dem Speicher-/Erhitzungs-Mittel mit einer Temperatur, die höher als 115 °C , vorzugsweise in der Größenordnung von 130 °C ist,
(ii) Umlauf des kalten thermischen Fluids in dem Bündel des zweiten Tauscher-Adsorbers (Ads2) in Adsorptionsphase gemäß Schritt 1), um das Aufrechterhalten der Kälte während Schritt 1) sicherzustellen,
progressives Erhitzen durch:
(iii) Austausch am Ende von Schritt 1) von kaltem thermischem Fluid, das in dem Bündel des zweiten Tauscher-Adsorbers (Ads2) vorhanden ist, mit dem lauwarmem thermischem Fluid, das in dem zweiten Speichermittel (S2) enthalten ist; das zweite Speichermittel (S2) enthält dann kaltes thermisches Fluid,
(iv) Erhitzen des thermischen Fluids, das in dem Bündel des zweiten Tauscher-Adsorbers (Ads2) enthalten ist, durch Wärmeaustausch über einen Tauscher (12) bis zu einer Temperatur von lauwarmem thermischem Fluid, die zwischen 80 °C und 105 °C, bevorzugt in der Größenordnung von 100 °C liegt,
(v) Austausch von lauwarmem thermischem Fluid, das in dem Bündel des zweiten Tauscher-Adsorbers (Ads2) am Ausgang von Schritt (iv) enthalten ist, mit dem heißen thermischen Fluid, das in dem ersten Speichermittel (S1) enthalten ist,
(vi) Erhitzen von heißem thermischem Fluid, das in dem Bündel des zweiten Tauscher-Adsorbers (Ads2) vorhanden ist, durch Umlauf von thermischem Fluid zwischen dem Bündel und dem Speicher-/Erhitzungs-Mittel (C) bis zum Ende der Regeneration gemäß Schritt 2).

4. Verfahren nach Anspruch 3, bei dem das progressive Abkühlen des ersten regenerierten Tauscher-Adsorbers (Ads1) am Ausgang von Schritt 2) und vor der Adsorption gemäß Schritt 1) mindestens die folgenden Schritte umfasst:
vor dem Abkühlen:
(vii) Bereitstellen:
o von heißem thermischem Fluid in dem Bündel des zweiten Tauscher-Adsorbers (Ads2) mit einer Temperatur des heißen Fluids, die höher als 110 °C ist, bevorzugt zwischen 115 °C und 130 °C beträgt,
o von thermischem Fluid in dem ersten Speichermittel (S1) mit einer Wassertemperatur in der Größenordnung von 100 °C bis 110 °C,
o von kaltem thermischem Fluid in dem zweiten Speichermittel (S2),
o von heißem thermischem Fluid in dem Speicher-/Erhitzungs-Mittel mit einer Temperatur, die höher als 115 °C , vorzugsweise in der Größenordnung von 130 °C ist,
und progressives Abkühlen, umfassend:
(viii) Austausch des thermischem Fluids, das in dem Bündel des zweiten Tauscher-Adsorbers (Ads2) vorhanden ist, mit dem thermischen Fluid des ersten Speichermittels (S1); dieses enthält dann heißes Wasser,
(ix) Austausch des lauwarmen Fluids, das in dem zweiten Tauscher-Adsorbers (Ads2) enthalten ist, mit dem kalten Fluid, das in dem zweiten Speichermittel (S2) enthalten ist,
(x) Erhitzen des Wassers, das in dem zweiten Speichermittel (S2) enthalten ist, durch ein Erhitzungsmittel HX, derart, dass das Speichern von lauwarmem Wasser gemäß Schritt (i) wiederhergestellt wird,
(xi) kontinuierliches Abkühlen von Ads2 durch kaltes thermisches Fluid.

5. Verfahren nach einem der Ansprüche 1 bis 4, bei dem das kalte Fluid dem allgemeinen Abkühlungssystem des Standorts entnommen wird.

6. Verfahren nach einem der vorhergehenden Ansprüche, **dadurch gekennzeichnet, dass** während Schritt 2) die Verunreinigungen dem Adsorptionsmittel durch Vakuumpumpen entzogen werden, derart, dass eine Druckmodulation einer Temperaturmodulation zugeordnet wird.

7. Verfahren nach Anspruch 6, bei dem ein Inertgas der Vakuumpumpe entgegengesetzt eingeleitet wird, derart, dass die Verunreinigungsfraktion in dem desorbierten Gas verdünnt und so dessen Teildruck verringert wird.

8. Verfahren nach Anspruch 6 oder Anspruch 7, bei dem die Adsorption bei einem Druck realisiert wird, der zwischen 7 und 15 bar beträgt, und das Wiederunterdrucksetzen des Tauscher-Adsorbers zur gleichen Zeit wie das Abkühlen realisiert wird.

9. Verfahren nach einem der Ansprüche 1 bis 8, bei dem das Speicher-/ Erhitzungs-Mittel (C) durch einen elektrischen Heizwiderstand erhitzt wird.

## Claims

1. Method for purifying, by adsorption, a feed flow rich in methane and containing at least carbon dioxide, using two exchanger-adsorbers (Ads1, Ads2) of the shell and tube type, comprising at least the steps of:
1) sending said feed flow to an exchanger-adsorber of the shell and tube type, having an adsorbent in the tubes and a cooling heat transfer fluid flowing in the shell of said exchanger-adsorber in order to produce a purified flow which has (at least) less carbon dioxide compared with the feed flow, then
2) passing a hot heat transfer fluid through the shell in order to desorb the impurities retained by the adsorbent and to regenerate said adsorbent,
in which steps 1) and 2) are carried out alternately on the two exchanger-adsorbers installed in parallel, the feed flow being sent to one of the exchanger-adsorbers while the second is in the regeneration phase, and then to the second while the first is in the regeneration phase, **characterised in that** it also comprises, at the start of step 2), a step of progressive heating of the exchanger-adsorber to be regenerated and, at the end of step 2), a step of progressive cooling of the regenerated exchanger-adsorber, said steps of progressive heating or cooling of the exchanger-adsorbers comprising at least exchanges of heat transfer fluid between the shell of the exchanger-adsorber, at least two storage means (S1, S2) which have a storage capacity comparable to the capacity of the shell and temporarily store the heat transfer fluid at variable temperatures, and a storage/heating means (C) which has a capacity greater than the capacity of the shell and is able to ensure additional heating of the heat transfer fluid for implementing step 2).

2. Method according to claim 1, wherein the heat transfer fluid is water.

3. Method according to either claim 1 or claim 2, wherein the progressive heating of the second exchanger-adsorber (Ads2) at the start of the step of regeneration comprises at least the steps of:
prior to heating:
(i) providing:
o cold heat transfer fluid in the shell of the exchanger-adsorber at a temperature lower than or approximately ambient temperature,
o hot heat transfer fluid in the first storage means (S1) at a temperature of greater than 110 °C,
o lukewarm heat transfer fluid in the second storage means (S2) at a temperature of between 70 °C and 110 °C, preferably of between 80 °C and 100 °C,
o hot heat transfer fluid in the storage/heating means at a temperature of greater than 115 °C, preferably of approximately 130 °C,
(ii) passing cold heat transfer fluid through the shell of the second exchanger-adsorber (Ads2) during the adsorption phase according to step 1) in order to ensure that the cold temperature is maintained during said step 1), and progressive heating by:
(iii) exchanging, at the end of step 1), cold heat transfer fluid present in the shell of the second exchanger-adsorber (Ads2) with lukewarm heat transfer fluid contained in the second storage means (S2); said second storage means (S2) thus now contains cold heat transfer fluid,
(iv) heating heat transfer fluid contained in the shell of the second exchanger-adsorber (Ads2) by means of heat exchange via an exchanger (12) up to a lukewarm heat transfer fluid temperature of between 80 °C and 105 °C,
preferably of approximately 100 °C,
(v) exchanging lukewarm heat transfer fluid contained in the shell of the second exchanger-adsorber (Ads2) at the end of step (iv) with the hot heat transfer fluid contained in the first storage means (S1),
(vi) heating hot heat transfer fluid present in the shell of the second exchanger-adsorber (Ads2) by means of passing heat transfer fluid between the shell and the storage/heating means (C) until the regeneration according to step 2) is completed.

4. Method according to claim 3, wherein the progressive cooling of the first regenerated exchanger-adsorber (Ads1) at the end of step 2) and prior to the adsorption according to step 1) comprises at least the steps of:
prior to cooling:
(vii) providing:
o hot heat transfer fluid in the shell of the second exchanger-adsorber (Ads2) at a hot fluid temperature of greater than 110 °C, preferably of between 115 °C and 130 °C,
o heat transfer fluid in the first storage means (S1) at a water temperature of from approximately 100 °C to 110 °C,
o cold heat transfer fluid in the second storage means (S2),
o hot heat transfer fluid in the storage/heating means (C) at a temperature of greater than 115 °C, preferably of approximately 130 °C,
and progressive cooling including:
(viii) exchanging heat transfer fluid present in the shell of the second exchanger-adsorber (Ads2) with the heat transfer fluid of the first storage means (S1); said first storage means (S1) thus now contains hot water,
(ix) exchanging lukewarm fluid contained in the second exchanger-adsorber (Ads2) with the cold fluid contained in the second storage means (S2),
(x) heating the water contained in the second storage means (S2) using a heating means HX in order to reconstitute the lukewarm water store according to step (i),
(xi) continuously cooling Ads2 using cold heat transfer fluid.

5. Method according to any of claims 1 to 4, wherein the cold fluid is taken from the general cooling system of the site.

6. Method according to any of the preceding claims, **characterised in that**, during step 2), impurities are removed from the adsorbent by means of vacuum pumping in order to associate a pressure modulation with the temperature modulation.

7. Method according to claim 6, wherein an inert gas is injected opposite the vacuum pump in order to dilute the proportion of impurities in the desorbed gas and to thus reduce the partial pressure thereof.

8. Method according to either claim 6 or claim 7, wherein adsorption is carried out at a pressure of between 7 and 15 bar and the repressurisation of the exchanger-adsorber is carried out at the same time as the cooling.

9. Method according to any of claims 1 to 8, wherein the storage/heating means (C) is heated by an electrical resistance heating means.
